(19) Europäisches Patentamt
European Patent Office
Office européen des brevets

(11) Veröffentlichungsnummer: **0 131 867**
**B1**

(12) # EUROPÄISCHE PATENTSCHRIFT

(45) Veröffentlichungstag der Patentschrift: **01.06.88**

(51) Int. Cl.[4]: **C 07 D 239/26**, A 01 N 43/54

(21) Anmeldenummer: **84107898.3**

(22) Anmeldetag: **06.07.84**

(54) **Aroxy-pyrimidinyl-alkanole.**

(30) Priorität: **16.07.83 DE 3325761**

(43) Veröffentlichungstag der Anmeldung:
**23.01.85 Patentblatt 85/4**

(45) Bekanntmachung des Hinweises auf die Patenterteilung:
**01.06.88 Patentblatt 88/22**

(84) Benannte Vertragsstaaten:
**AT BE CH DE FR GB IT LI NL SE**

(56) Entgegenhaltungen:
**EP-A-0 001 399**
**EP-A-0 028 755**

(73) Patentinhaber: **BAYER AG, Konzernverwaltung RP Patentabteilung, D-5090 Leverkusen 1 Bayerwerk (DE)**

(72) Erfinder: **Holmwood, Graham, Dr., Krutscheider Weg 105, D 5600 Wuppertal 11 (DE)**
Erfinder: **Lürssen, Klaus, Dr., August-Kierspel-Strasse 151, D 5060 Bergisch Gladbach 2 (DE)**
Erfinder: **Reinecke, Paul, Dr., Lessingstrasse 11, D 5090 Leverkusen 3 (DE)**
Erfinder: **Brandes, Wilhelm, Dr., Eichendorffstrasse 3, D 5653 Leichlingen 1 (DE)**
Erfinder: **Hänssler, Gerd, Dr., Heymannstrasse 40, D 5090 Leverkusen (DE)**

EP 0 131 867 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Aroxy-pyrimidinyl-alkanole, ein Verfahren zu ihrer Herstellung und ihre Verwendung als Pflanzenwachstumsregulatoren und Fungizide.

Es ist bereits bekannt geworden, dass bestimmte Phenoxy-pyrimidinyl-alkanole pflanzenwachstumsregulierende und fungizide Eigenschaften besitzen (vgl. DE-A 2 742 173 und DE-A 2 944 850). So lässt sich zum Beispiel das 3,3-Dimethyl-1-(4-chlor-phenoxy)-2-(pyrimidin-5-yl)-butan-2-ol zur Regulierung des Pflanzenwachstums und zur Bekämpfung von Pilzen einsetzen. Die Wirkung dieses Stoffes ist gut, lässt jedoch bei niedrigen Aufwandmengen in manchen Fällen zu wünschen übrig.

Es wurden nun neue Aroxy-pyrimidinyl-alkanole der Formel

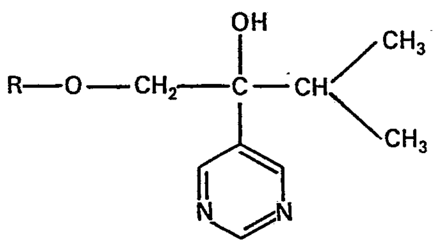

(I)

in welcher

R für gegebenenfalls durch Halogen substituiertes Naphthyl oder für den Rest der Formel

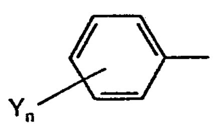

steht, worin

Y für Fluor, Chlor, Brom, Alkyl mit 1 bis 4 Kohlenstoffatomen, Cycloalkyl mit 5 bis 7 Kohlenstoffatomen, Alkoxy mit 1 bis 4 Kohlenstoffatomen, Halogenalkyl mit 1 oder 2 Kohlenstoffatomen und 1 bis 5 Halogenatomen Halogenalkoxy mit 1 oder 2 Kohlenstoffatomen und 1 bis 5 Halogenatomen, Alkylthio mit 1 bis 4 Kohlenstoffatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkoxy mit 1 bis 4 Kohlenstoffatomen im Alkoxyteil, gegebenfalls durch Halogen und/oder Alkyl mit 1 bis 4 Kohlenstoffatomen substituiertes Phenylalkyl mit 1 bis 4 Kohlenstoffatomen im Alkylteil steht, und der Index

n für 0, 1, 2 oder 3 steht, wobei die durch das Symbol Y bezeichneten Substituierten gleich oder verschieden sein können, sowie deren Säureadditions-Salze und Metallsalz-Komplexe gefunden.

Die Aroxy-pyrimidinyl-alkanole enthalten ein asymmetrisch substituiertes Kohlenstoffatom und können deshalb in den beiden optisch-isomeren Formen oder als Racemate vorliegen. Die Erfindung betrifft sowohl die Isomeren-Gemische als auch die einzelnen Isomeren.

Weiterhin wurde gefunden, dass man die Aroxy-pyrimidinyl-alkanole der Formel (I) sowie deren Säureadditions-Salze und Metallsalz-Komplexe erhält, wenn man Aroxyketone der Formel

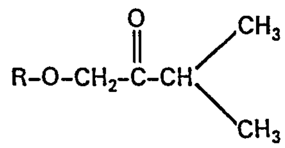

(II)

in welcher

R die oben angegebene Bedeutung hat, mit Pyrimidinyl-halogeniden der Formel

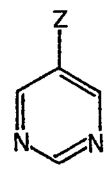

(III)

in welcher

Z für Halogen steht, in Gegenwart eines Verdünnungsmittels und in Gegenwart einer alkalimetall-organischen Verbindung umsetzt und gegebenenfalls anschliessend eine Säure oder ein Metallsalz addiert.

Schliesslich wurde gefunden, dass die neuen Aroxy-pyrimidinyl-alkanole der Formel (I) sehr gute pflanzenwuchsregulierende und fungizide Eigenschaften besitzen.

Es ist als äusserst überraschend zu bezeichnen, dass die erfindungsgemässen Aroxy-pyrimidinyl-alkanole der Formel (I) eine bessere pflanzenwuchsregulierende und fungizide Wirkung zeigen als das 3,3-Dimethyl-1-(4-chlor-phenoxy)-2-(pyrimidin-5-yl)-butan-2-ol, welches der konstitutionell ähnlichste vorbekannte Wirkstoff gleicher Wirkungsrichtung ist.

Bevorzugt sind diejenigen Verbindungen der Formel (I), in denen R für gegebenenfalls durch Fluor, Chlor und/oder Brom substituiertes Naphth-1-yl oder für den Rest der Formel

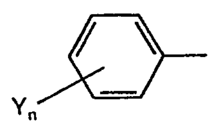

steht, worin Y für Fluor, Chlor, Brom, Methyl, Ethyl, Isopropyl, n-Propyl, Cyclohexyl, Methoxy, Ethoxy, n-Propoxy, Isopropoxy, Halogenalkyl mit 1 oder 2 Kohlenstoffatomen und 1 bis 3 Fluor- und/oder Chloratomen, Halogenalkoxy mit 1 oder

2 Kohlenstoffatomen und 1 bis 3 Fluor- und/oder Chloratomen, Methylthio, Ethylthio, gegebenenfalls durch Fluor, Chlor, Methyl und/oder Ethyl substituiertes Phenyl, gegebenenfalls durch Fluor, Chlor, Methyl und/oder Ethyl substituiertes Phenoxy, gegebenenfalls durch Fluor, Chlor, Methyl und/oder Ethyl substituiertes Phenylalkyl mit 1 oder 2 Kohlenstoffatomen in der Alkylengruppe, gegebenenfalls durch Fluor, Chlor, Methyl und/oder Ethyl substituiertes Phenylakoxy mit 1 oder 2 Kohlenstoffatomen in der Alkoxygruppe steht, und der Index n für 0, 1, 2 oder 3 steht.

Bevorzugte erfindungsgemässe Stoffe sind auch Säureadditions-Salze von Aroxy-pyrimidinyl-alkanolen der Formel (I), in denen R die oben angegebenen vorzugsweisen Bedeutungen hat. Besonders bevorzugt sind dabei diejenigen Salze, die durch Addition der folgenden Säuren entstehen: Chlorwasserstoffsäure, Bromwasserstoffsäure, Phosphorsäure, Salpetersäure, Schwefelsäure, p-Toluolsulfonsäure oder 1,5-Naphthalin-disulfonsäure.

Bevorzugte erfindungsgemässe Stoffe sind ausserdem Metallsalz-Komplexe von Aroxy-pyrimidinyl-alkanolen der Formel (I), in denen R die oben angegebenen vorzugsweisen Bedeutungen hat. Besonders bevorzugt sind dabei diejenigen Komplexe, die Salze von Metallen der II. bis IV. Hauptgruppe und der I. und II. sowie IV. bis VIII. Nebengruppe des Periodensystems enthalten, wobei Kupfer, Zink, Mangan, Magnesium, Zinn, Eisen und Nickel beispielhaft genannt seien.

Verwendet man 1-(4-Chlor-phenoxy)-3-methyl-butan-2-on und 5-Brom-pyrimidin als Ausgangsstoffe und n-Butyl-lithium als alkalimetallorganische Verbindung, so kann der Ablauf des erfindungsgemässen Verfahrens durch das folgende Formelschema wiedergegeben werden:

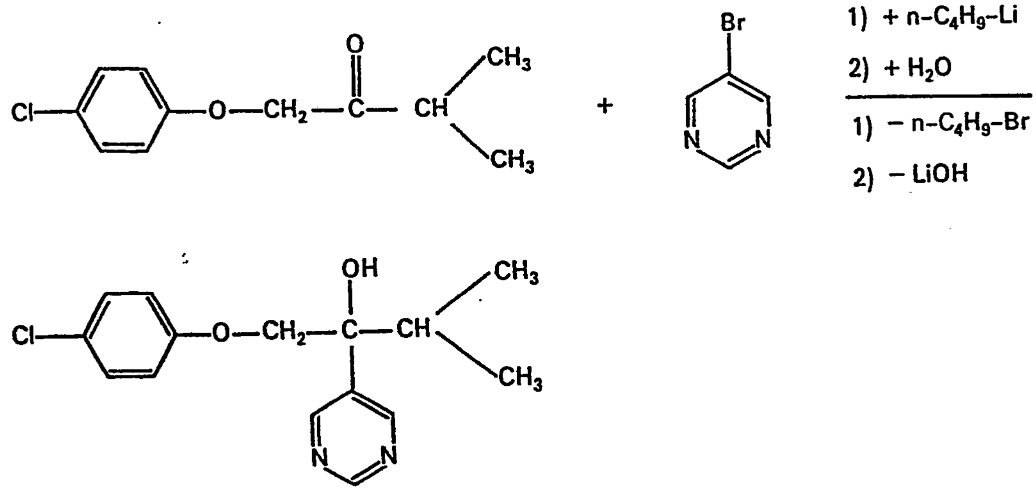


Die bei dem erfindungsgemässen Verfahren als Ausgangsstoffe benötigten Aroxyketone sind durch die Formel (II) allgemein definiert. In dieser Formel steht R vorzugsweise für diejenigen Reste, die bereits im Zusammenhang mit der Beschreibung der erfindungsgemässen Stoffe der Formel (I) vorzugsweise für diesen Substituenten genannt wurden.

Die Aroxyketone der Formel (II) sind bekannt oder lassen sich nach bekannten Verfahren in einfacher Weise herstellen (vgl. DE-A 2 105 490, DE-A 2 201 063 und Org. Synth. 55, 24). Man erhält die Aroxyketone zum Beispiel, indem man entsprechende Arylhydroxy-Verbindungen mit entsprechenden Halogenketonen in Gegenwart eines Säurebindemittels sowie in Gegenwart eines Verdünnungsmittels umsetzt.

Die bei dem erfindungsgemässen Verfahren weiterhin als Ausgangsstoffe benötigten Pyrimidinyl-halogenide sind durch die Formel (III) allgemein definiert. In dieser Formel steht Z vorzugsweise für Chlor oder Brom.

Die Pyrimidinyl-halogenide der Formel (III) sind allgemein bekannte Verbindungen der organischen Chemie.

Als Verdünnungsmittel kommen bei dem erfindungsgemässen Verfahren alle inerten organischen Solventien in Frage. Vorzugsweise verwendbar sind solche organischen Lösungsmittel, die einen tiefen Festpunkt besitzen, wie zum Beispiel Ether. Besonders bevorzugt sind dabei Diethylether und Tetrahydrofuran sowie auch Gemische aus diesen beiden Solventien.

Als alkalimetallorganische Verbindungen können bei der erfindungsgemässen Umsetzung vorzugsweise Alkalimetall-Alkyle, wie zum Beispiel n-Butyl-lithium, oder Alkalimetall-Aryle, wie zum Beispiel Phenyl-lithium, verwendet werden.

Die Reaktionstemperaturen können bei dem erfindungsgemässen Verfahren innerhalb eines bestimmten Bereiches variiert werden. Im allgemeinen arbeitet man bei Temperaturen zwischen -150°C und -50°C, vorzugsweise zwischen -120°C und -80°C.

Die erfindungsgemässe Umsetzung wird vorzugsweise unter Inertgas-Atmosphäre, wie zum Beispiel Stickstoff oder Argon, vorgenommen.

Bei der Durchführung des erfindungsgemässen Verfahrens setzt man die Aroxyketone der Formel (II) und die Pyrimidinyl-halogenide der Formel (III) im allgemeinen in angenähert äquimolaren Mengen ein. Es ist jedoch auch möglich, eine der beiden Komponenten in einem grösseren Überschuss zu verwenden. Die alkalimetall-

organische Verbindung wird im allgemeinen in einem Überschuss von 5 bis 75 Mol-%, vorzugsweise von 10 bis 50 Mol-%, eingesetzt. Es kann so verfahren werden, dass man zunächst die alkalimetall-organische Verbindung mit dem Pyrimidinyl-halogenid der Formel (III) reagieren lässt und anschliessend das Aroxyketon der Formel (II) zufügt; man kann aber auch das Aroxyketon der Formel (II) und das Pyrimidinyl-halogenid der Formel (III) vorlegen und anschliessend bei tiefer Temperatur, zum Beispiel bei -100°C bis -130°C, die alkalimetall-organische Verbindung zugeben.

Die Isolierung der Aroxy-pyrimidinyl-alkanole der Formel (I) erfolgt im allgemeinen in der Weise, dass man das bei der Umsetzung zunächst gebildete Alkali-alkanolat, zum Beispiel Lithiumalkanolat, mit Wasser hydrolysiert. Die weitere Aufarbeitung erfolgt nach üblichen Methoden.

Zur Herstellung von Säureadditions-Salzen der Verbindungen der Formel (I) kommen alle diejenigen Säuren in Frage, die zu physiologisch verträglichen Salzen führen. Vorzugsweise verwendbar sind diejenigen Säuren, die bereits im Zusammenhang mit der Beschreibung der erfindungsgemässen Stoffe als bevorzugt zu addierende Säuren genannt wurden.

Die Säureadditions-Salze der Verbindungen der Formel (I) lassen sich in einfacher Weise nach üblichen Salzbildungsmethoden herstellen. Im allgemeinen verfährt man so, dass man eine Verbindung der Formel (I) in einem geeigneten inerten Verdünnungsmittel löst und dann eine Säure hinzufügt. Die Isolierung erfolgt in bekannter Weise, zum Beispiel dadurch, dass man das Salz abfiltriert und gegebenenfalls durch Waschen mit einem inerten organischen Lösungsmittel reinigt.

Zur Herstellung von Metallsalz-Komplexen der Verbindungen der Formel (I) kommen vorzugsweise Salze von denjenigen Metallen in Betracht, die bereits im Zusammenhang mit der Beschreibung der erfindungsgemässen Stoffe als bevorzugt zu addierende Metalle genannt wurden. Als Anionen dieser Metallsalze kommen vorzugsweise Anionen der Halogenwasserstoffsäuren, wie zum Beispiel Chlorwasserstoffsäure und Bromwasserstoffsäure, ferner Phosphorsäure, Salpetersäure und Schwefelsäure in Frage.

Die Metallsalz-Komplexe von Verbindungen der Formel (I) lassen sich in einfacher Weise nach üblichen Methoden herstellen. Im allgemeinen geht man so vor, dass man ein Metallsalz in Alkohol, wie zum Beispiel Ethanol, löst und dann eine Verbindung der Formel (I) hinzufügt. Die Isolierung erfolgt ebenfalls in bekannter Weise, zum Beispiel dadurch, dass man den Metallsalz-Komplex abfiltriert und gegebenenfalls durch Umkristallisation reinigt.

Die erfindungsgemässen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Strassenrändern, auf Flughäfen oder in Obstanlagen reduziert werden. Von Bedeutung ist auch die Hemmung des Wuchses von krautigen und holzigen Pflanzen an Strassenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefahr besteht, dass das Getreide lagert.

Eine Hemmung des vegetativen Wachstums ermöglicht bei vielen Kulturpflanzen eine dichtere Anpflanzung, so dass Mehrerträge bezogen auf die Bodenfläche erzielt werden können. Ein Vorteil der so erzielten kleineren Pflanzen ist auch, dass die Kultur leicht bearbeitet und beerntet werden kann.

Eine Hemmung des vegetativen Wachstums der Pflanzen kann auch dadurch zu Ertragssteigerungen führen, dass die Nährstoffe und Assimilate in stärkerem Masse der Blüten- und Fruchtbildung zugute kommen als den vegetativen Pflanzenteilen.

Mit Wachstumsregulatoren lässt sich häufig auch eine Förderung des vegetativen Wachstums erzielen. Die ist von grossem Nutzen, wenn die vegetativen Pflanzenteile geerntet werden. Eine Förderung des vegetativen Wachstums kann aber auch gleichzeitig zu einer Förderung des generativen Wachstums führen, dadurch dass mehr Assimilate gebildet werden, so dass mehr oder grössere Früchte entstehen.

Ertragssteigerungen können in manchen Fällen durch einen Eingriff in den pflanzlichen Stoffwechsel erreicht werden, ohne dass sich Änderungen des vegetativen Wachstums bemerkbar machen. Ferner kann mit Wachstumsregulatoren eine Veränderung der Zusammensetzung der

Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Ausserdem lässt sich die Produktion oder der Abfluss von sekundären Pflanzeninhaltsstoffen positiv beeinflussen. Als Beispiel sei die Stimulierung des Latexflusses bei Gummibäumen genannt.

Unter dem Einfluss von Wachstumsregulatoren kann es zur Ausbildung parthenokarper Früchte kommen. Ferner kann das Geschlecht der Blüten beeinflusst werden. Auch kann eine Sterilität des Pollens erzeugt werden, was bei der Züchtung und Herstellung von Hybridsaatgut eine grosse Bedeutung hat.

Durch den Einsatz von Wachstumsregulatoren lässt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. grosses Interesse im Tabakanbau oder bei der Anpflanzung von Tomaten.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Baumwolle eine grosse Rolle, ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden («Ausdünnung»), um die Alternanz zu brechen. Unter Alternanz versteht man die Eigenart einiger Obstarten, endogen bedingt von Jahr zu Jahr sehr unterschiedliche Erträge zu bringen. Schliesslich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren lässt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, dass z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schliesslich kann mit Wachstumsregulatoren eine Resistenz der Pflanzen gegen Frost, Trokkenheit oder hohen Salzgehalt des Bodens indiziert werden. Hierdurch wird die Kultivierung von Pflanzen in Gebieten möglich, die hierzu normalerweise ungeeignet sind.

Die erfindungsgemässen Wirkstoffe eignen sich besonders gut zur Hemmung des Längenwachstums der Pflanzen, insbesondere von Getreide, wie zum Beispiel Gerste, Weizen, Roggen, Hafer und Reis.

Die erfindungsgemässen Wirkstoffe weisen auch eine starke fungizide und mikrobizide Wirkung auf und können deshalb zur Bekämpfung von unerwünschten Pilzen und Mikroorganismen eingesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der erfindungsgemässen Wirkstoffe in den zur Bekämpfung von Pflanzenkrankheiten notwendigen Konzentraten erlaubt eine Behandlung von oberirdischen Pflanzenteilen, von Pflanz- und Saatgut und des Bodens.

Als fungizide Mittel können die erfindungsgemässen Wirkstoffe mit besonders gutem Erfolg zur Bekämpfung solcher Pilze eingesetzt werden, die echte Mehltau-Erkrankungen hervorrufen; zum Beispiel zur Bekämpfung von Erysiphe-Arten, wie gegen den Erreger des Gersten- bzw. Getreidemehltaus (Erysiphe graminis). Ferner zeigen sie besonders gute Erfolge gegen den Erreger des Apfelschorfes (Venturia inaequalis) sowie gegen Pyricularia oryzae an Reis.

Besonders hervorzuheben ist, dass die erfindungsgemässen Wirkstoffe nicht nur eine protektive Wirkung entfalten, sondern auch systemisch wirksam sind. So gelingt es, Pflanzen gegen Pilzfall zu schützen, wenn man den Wirkstoff über den Boden und die Wurzeln oder über das Saatgut den oberirdischen Teilen der Pflanzen

zuführt. Ausserdem zeigen die erfindungsgemässen Stoffe eine gute fungizide Wirkung gegen Fusarien, Septoria, Cochliobolus sativus und Pyrenophora teres an Getreide. Die erfindungsgemässen Stoffe besitzen darüber hinaus auch gute herbizide Eigenschaften.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethysulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstofe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-Ether, Alkylsulfonate, Alkylsulfate, Arylsulfonate sowie Eiweisshydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetate.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Giessen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei Einsatz der erfindungsgemässen Wirkstoffe als Pflanzenwachstumsregulatoren können die Aufwandmengen in einem grösseren Bereich variiert werden. Im allgemeinen verwendet man pro Hektar Bodenfläche 0,001 bis 5 kg, bevorzugt 0,005 bis 1 kg an Wirkstoff.

Für den Einsatz der erfindungsgemässen Stoffe als Pflanzenwachstumsregulatoren gilt, dass die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemässen Stoffe als Fungizide kann die Aufwandmenge je nach Art der Applikation in einem grösseren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Herstellung und die Verwendung der erfindungsgemässen Wirkstoffe geht aus den folgenden Beispielen hervor.

Herstellungsbeispiele

Beispiel 1

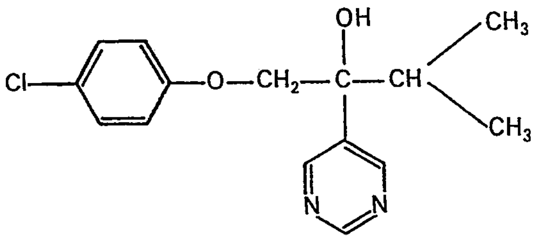

In einer Lösung von 206,7 g (1,3 Mol) 5-Brompyrimidin in 2 Litern eines Gemisches, das zu gleichen Teilen aus absolutem Diethylether und absolutem Tetrahydrofuran bestand, wurden bei -115°C bis -120°C 250 ml einer 50%igen Lösung von n-Butyllithium in Hexan unter Rühren eingetropft. Nach einstündigem Nachrühren bei -115° bis -120°C wurde bei gleicher Temperatur eine Lösung von 282 g (1,3 Mol) 1-(4-Chlorphenoxy)-3-methyl-butan-2-on in 500 ml absolutem Tetrahydrofuran eingetropft. Man rührte das Reaktionsgemisch noch 2 Stunden bei -115°C bis -120°C, ersetzte dann das Kühlbad durch ein Aceton/Trockeneis-Kühlbad und rührte weitere 16 Stunden. Danach wurde eine Lösung von 80,3 g (1,5 Mol) Ammoniumchlorid in wenig Wasser eingetropft. Nach dem Abtrennen der wässrigen Phase wurde die organische Phase nacheinander einmal mit halbgesättigter und zweimal mit gesättigter wässriger Kochsalz-Lösung gewaschen, dann über Natriumsulfat getrocknet und durch Abziehen des Lösungsmittels unter vermindertem Druck eingeengt. Der verbleibende Rückstand wurde in 1,5 Litern Toluol gelöst und nacheinander mit 320 ml und dann mit 40 ml halbkonzentrierter wässriger Salzsäure extrahiert. Anschliessend überschichtete man den Salzsäure-Extrakt mit 1 Liter Toluol, stellte dann durch langsame Zugabe von 45%iger wässriger Natronlauge unter Kühlung basisch, trennte die wässrige Phase ab und extrahierte nochmals mit Toluol. Nach dem Abziehen des Lösungsmittels unter vermindertem Druck verblieb ein hell-braunes Öl, das allmählich durchkristallisierte. Man erhielt auf diese Weise 254 g (66,8% der Theorie) an 1-(4-Chlor-phenoxy)-3-methyl-2-(pyrimidin-5-yl)-butan-2-ol in Form einer Testsubstanz vom Schmelzpunkt 77-78°C.

Herstellung des Ausgangsproduktes der Formel

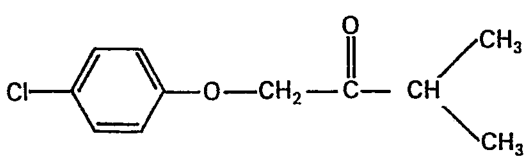

In eine Mischung aus 357 g (2,78 Mol) 4-Chlorphenol und 483 g (3,5 Mol) Kaliumcarbonat in 2,5 Litern Methyl-ethyl-keton wurden unter Rühren 338 g (2,77 Mol) 1-Brom-3-methyl-butan-2-on schnell eingetropft. Das Gemisch wurde 16 Stunden unter Rückfluss erhitzt und dann filtriert. Man engte das Filtrat durch Abziehen des Lösungsmittels unter vermindertem Druck ein, nahm den Rückstand in Methylenchlorid auf, wusch die organische Phase zweimal mit 2 n wässriger Natronlauge, einmal mit Wasser und einmal mit gesättigter wässriger Kochsalzlösung. Nach dem Trocknen wurde die organische Phase durch Abziehen des Lösungsmittels eingeengt. Der verbleibende Rückstand wurde im Hochvakuum destilliert. Man erhielt auf diese Weise 435 g (74% der Theorie) an 1-(4-Chlorphenoxy)-3-methyl-butan-2-on. Kp = 103-105°C/0,15 mbar.

Nach der im Beispiel 1 angegebenen Methode lassen sich auch die in den folgenden Beispielen formelmässig aufgeführten erfindungsgemässen Stoffe herstellen.

Tabelle 1

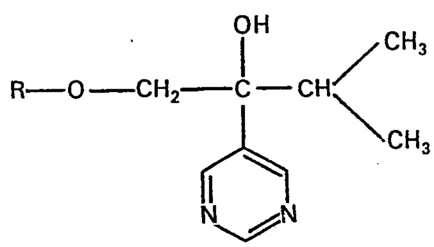 (I)

| Beispiel-Nr. | R | Schmelzpunkt [°C] oder Brechungsindex [$n_D^{20}$] |
|---|---|---|
| 2 | 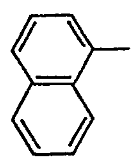 | 96-98 |
| 3 | 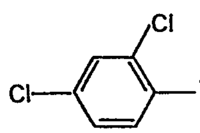 | 127-128 |
| 4 | 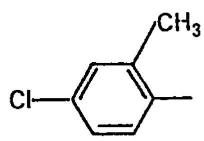 | 117-118 |
| 5 | 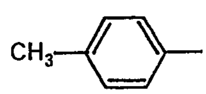 | 1,5497 |
| 6 | 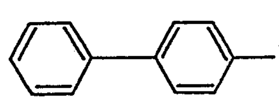 | 157-158 |

Tabelle 1 (Fortsetzung)

| Beispiel-Nr. | R | Schmelzpunkt [°C] oder Brechungsindex [$n_D^{20}$] |
|---|---|---|
| 7 | 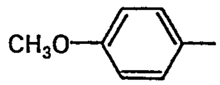 | 95–96 |
| 8 | 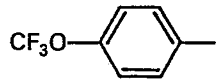 | 1,5081 |
| 9 | 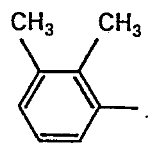 | 98–99 |
| 10 | 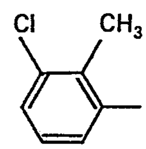 | 87–88 |
| 11 | 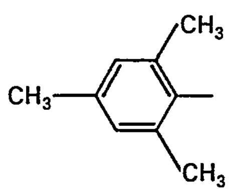 | 93–94 |

In den folgenden Verwendungsbeispielen wurden die nachstehend angegebenen Substanzen als Vergleichskomponenten eingesetzt:

A 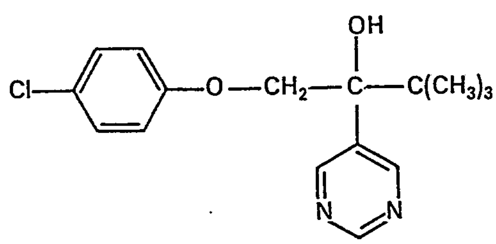

[3,3-Dimethyl-1-(4-chlor-phenoxy)-2-(pyrimidin-5-yl)-butan-2-ol; bekannt aus DE-OS 2 742 173]

B 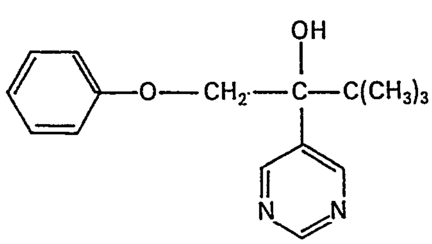

[3,3-Dimethyl-1-phenoxy-2-(pyrimidin-5-yl)-butan-2-ol; bekannt aus DE-OS 2 742 173].

C 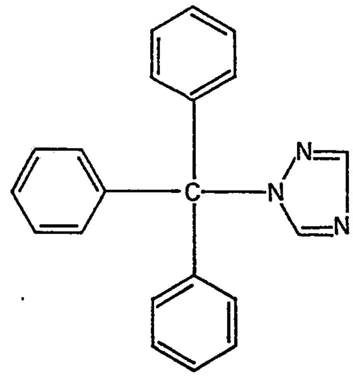

D 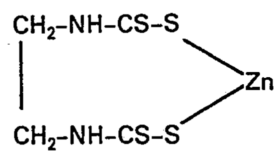

Beispiel A

Wuchshemmung bei Sojabohnen

Lösungsmittel: 30 Gew.-Teile Dimethylformamid

Emulgator: 1 Gew.-Teil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässe Verbindung (1) zeigte in diesem Test sowohl bei einer Konzentration von 0,05% als auch bei einer Konzentration von 0,0125% eine wesentlich stärkere wuchshemmende Wirkung als die Vergleichssubstanz (A).

Tabelle A
Wuchshemmung bei Sojabohnen

| Wirkstoff | Konzentration [in %] | Wuchshemmung [in %] |
|---|---|---|
| – (Kontrolle) | – | 0 |
| (A) | 0,05 | 78 |
| bekannt | 0,0125 | 65 |
| (1) | 0,05 | 91 |
| | 0,0125 | 88 |
| (3) | 0,05 | 82 |
| (5) | 0,05 | 89 |
| (7) | 0,05 | 89 |
| (8) | 0,05 | 86 |
| (11) | 0,05 | 89 |

Beispiel B

Wuchshemmung bei Gerste

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blatt-Stadium angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässe Verbindung (1) zeigte in diesem Test bei einer Konzentration von 0,05% eine deutlich stärkere wuchshemmende Wirkung als die Vergleichssubstanz (A). Bei niedrigeren Konzentrationen als 0,05% wurde das Wachstum durch die Verbindung (A) nicht mehr gehemmt, während die erfindungsgemässe Verbindung (1) noch einen deutlichen Effekt ausübte.

Tabelle B

Wuchshemmung bei Gerste

| Wirkstoff | Konzentration [in %] | Wuchshemmung [in %] |
|---|---|---|
| – (Kontrolle) | – | 0 |
| (A) | 0,05 | 49 |
| bekannt | 0,025 | 0 |
| | 0,0125 | 0 |
| (1) | 0,05 | 64 |
| | 0,0125 | 38 |

Beispiel C

Puccinia-Test (Weizen)/protektiv

Lösungsmittel: 100 Gew.-Teile Dimethylformamid

Emulgator: 0,25 Gew.-Teile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1%igen wässrigen Agarlösung inokuliert. Nach Antrocknen besprüht man die Pflanzen mit der Wirkstoffzubereitung taufeucht. Die Pflanzen verbleiben 24 Stunden bei 20°C und 100% relativer Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt, um die Entwicklung von Rostpusteln zu begünstigen.

Die Auswertung erfolgte 10 Tage nach der Inokulation.

Die erfindungsgemässe Verbindung (1) zeigte in diesem Test bei einer Konzentration von 0,025% eine deutlich bessere Wirkung als die Vergleichssubstanz (A).

Tabelle C

Puccinia-Test (Weizen)/protektiv

| Wirkstoff | Wirkstoffkonzentration in der Spritzbrühe in Gew.% | Krankheitsbefall in % der unbehandelten Kontrolle |
|---|---|---|
| bekannt | | |
| 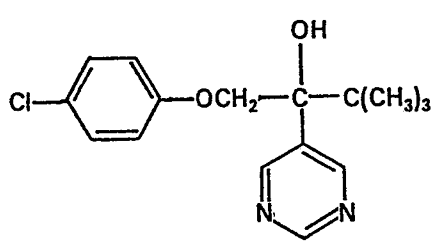 (A) | 0,025 | 72,5 |

Tabelle C (Fortsetzung)

| Wirkstoff | Wirkstoffkonzentration in der Spritzbrühe in Gew.% | Krankheitsbefall in % der unbehandelten Kontrolle |
|---|---|---|
| erfindungsgemäss: | | |
| 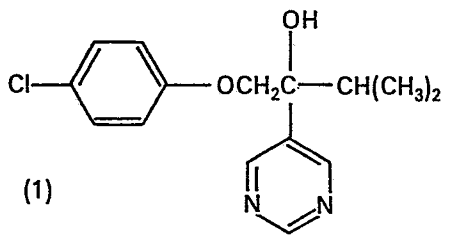 (1) | 0,025 | 25,0 |

Beispiel D

Erysiphe-Test (Gerste)/protektiv

Lösungsmittel: 100 Gew.-Teile Dimethylformamid

Emulgator: 0,25 Gew.-Teile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgte die Auswertung.

Die erfindungsgemässe Verbindung (1) zeigte in diesem Test bei einer Konzentration von 0,00025% eine deutlich bessere Wirkung als die Vergleichssubstanz (B).

Tabelle D
Erysiphe-Test (Gerste)/protektiv

| Wirkstoff | Wirkstoffkonzentration in der Spritzbrühe in Gew.% | Krankheitsbefall in % der unbehandelten Kontrolle |
|---|---|---|
| bekannt: | | |
| 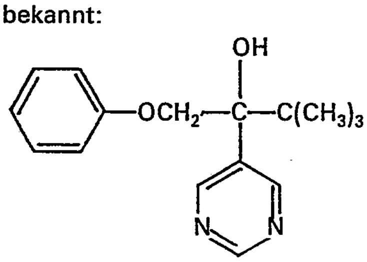 (B) | 0,00025 | 100 |
| erfindungsgemäss: | | |
| 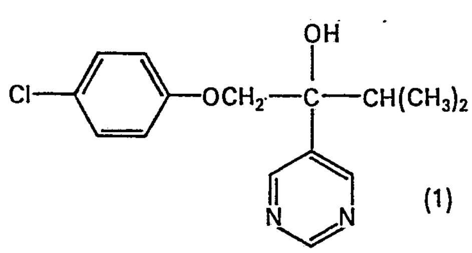 (1) | 0,00025 | 48,7 |

Beispiel E

Venturia-Test (Apfel)/protektiv

Lösungsmittel: 4,7 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Konidiensuspension des Apfelschorferregers (Venturia inaequalis) inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgte die Auswertung.

Wirkstoffe, Wirkstoffkonzentrationen und Versuchsergebnisse gehen aus der folgenden Tabelle hervor.

Tabelle E

Venturia-Test (Apfel)/protektiv

| Wirkstoff | Befall in % bei einer Wirkstoffkonzentration von 25 ppm | 10 ppm |
|---|---|---|
| bekannt: | | |
| 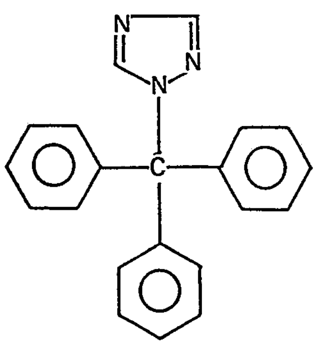 | 82 | |
| erfindungsgemäss: | | |
| 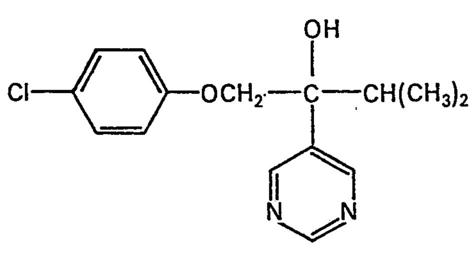 | | 20 |

Beispiel F

Pyricularia-Test (Reis)/protektiv

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wässrigen Sporensuspension von Pyricularia oryzae inokuliert. Anschliessend werden die Pflanzen in einem Gewächshaus bei 100% rel. Luftfeuchtigkeit und 25°C aufgestellt.

4 Tage nach der Inokulation erfolgte die Auswertung des Krankheitsbefalls.

Wirkstoffe, Wirkstoffkonzentrationen und Versuchsergebnisse gehen aus der folgenden Tabelle hervor.

Tabelle F
Pyricularia-Test (Reis)/protektiv

| Wirkstoffe | Wirkstoffkonzentration in % | Krankheitsbefall in % der unbehandelten Kontrolle |
|---|---|---|
| bekannt: | | |
| 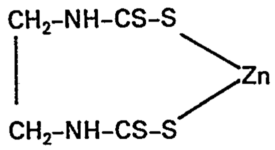 | 0,025 | 75 |
| erfindungsgemäss: | | |
| 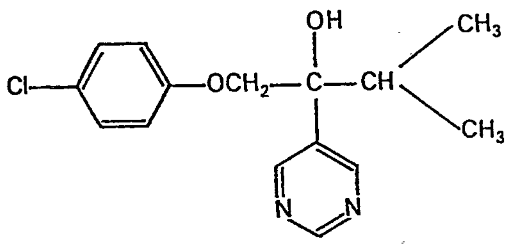 | 0,025 | 20 |

Beispiel G

Wuchshemmung bei Gras (Festuca pratensis)

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnass besprüht. Nach 3 Wochen wird der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Wirkstoffe, Wirkstoffkonzentrationen und Versuchsergebnisse gehen aus der folgenden Tabelle hervor.

Tabelle G
Wuchshemmung bei Gras (Festuca pratensis)

| Wirkstoff | Wirkstoffkonzentration in % | Wuchshemmung in % |
|---|---|---|
| – (Kontrolle) | – | = 0 |
| (4) | 0,05 | 76 |
| (5) | 0,05 | 66 |

Tabelle G
Wuchshemmung bei Gras (Festuca pratensis)

| Wirkstoff | Wirkstoffkonzentration in % | Wuchshemmung in % |
|---|---|---|
| (8) | 0,05 | 91 |
| (9) | 0,05 | 66 |

Beispiel H

Wuchshemmung bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnass mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Wirkstoffe, Wirkstoffkonzentrationen und Versuchsergebnisse gehen aus der folgenden Tabelle hervor.

Tabelle H
Wuchshemmung bei Baumwolle

| Wirkstoff | Wirkstoff-konzentration in % | Wuchs-hemmung in % |
|---|---|---|
| – Kontrolle | – | = 0 |
| (4) | 0,05 | 100 |
| (3) | 0,05 | 82 |
| (5) | 0,05 | 76 |
| (7) | 0,05 | 76 |
| (8) | 0,05 | 80 |
| (10) | 0,05 | 100 |
| (11) | 0,05 | 76 |

**Patentansprüche**

1. Aroxy-pyrimidinyl-alkanole der Formel

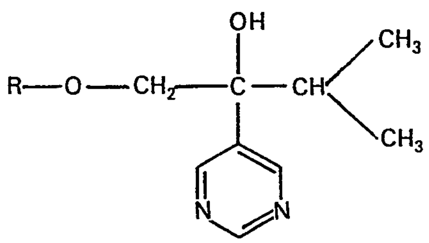

(I)

in welcher
R für gegebenenfalls durch Halogen substituiertes Naphthyl oder für den Rest der Formel

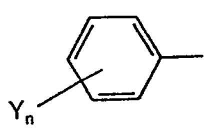

steht, worin
Y für Fluor, Chlor, Brom, Alkyl mit 1 bis 4 Kohlenstoffatomen, Cycloalkyl mit 5 bis 7 Kohlenstoffatomen, Alkoxy mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Alkylthio mit 1 bis 4 Kohlenstoffatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkoxy mit 1 bis 4 Kohlenstoffatomen im Alkoxyteil, gegebenenfalls durch Halogen und/oder Alkyl mit 1 bis 4 Kohlenstoffatomen substituiertes Phenylalkyl mit 1 bis 4 Kohlenstoffatomen im Alkylteil steht, und der Index

n für 0, 1, 2 oder 3 steht,

wobei die durch das Symbol Y bezeichneten Substituierten gleich oder verschieden sein können, sowie deren Säureadditions-Salze und Metallsalz-Komplexe.

2. Aroxy-pyrimidinyl-alkanol der Formel

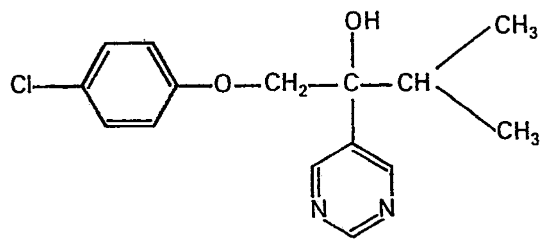

3. Aroxy-pyrimidinyl-alkanol der Formel

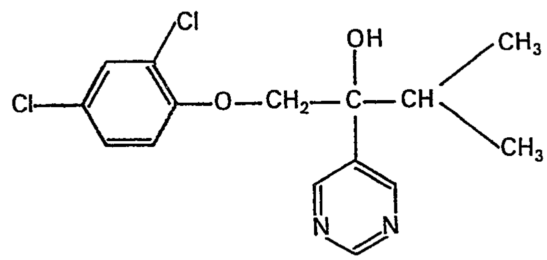

4. Aroxy-pyrimidinyl-alkanol der Formel

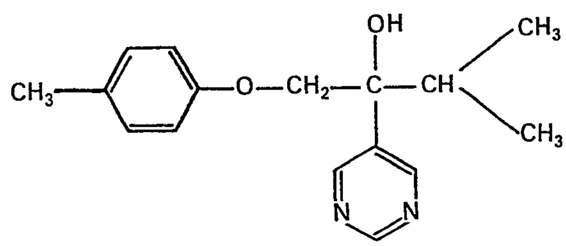

5. Verfahren zur Herstellung von Aroxy-pyrimidinyl-alkanolen der Formel

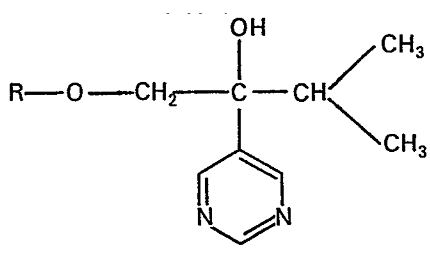

(I)

in welcher
für gegebenenfalls durch Halogen substituiertes Naphthyl oder für den Rest der Formel

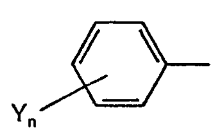

Y für Fluor, Chlor, Brom, Alkyl mit 1 bis 4 Kohlenstoffatomen, Cycloalkyl mit 5 bis 7 Kohlenstoffatomen, Alkoxy mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen,

Alkylthio mit 1 bis 4 Kohlenstoffatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkoxy mit 1 bis 4 Kohlenstoffatomen im Alkoxyteil, gegebenenfalls durch Halogen und/oder Alkyl mit 1 bis 4 Kohlenstoffatomen substituiertes Phenylalkyl mit 1 bis 4 Kohlenstoffatomen im Alkylteil steht, und der Index

n für 0, 1, 2 oder 3 steht,

wobei die durch das Symbol Y bezeichneten Substituierten gleich oder verschieden sein können, sowie von deren Säureadditions-Salzen und Metallsalz-Komplexen, dadurch gekennzeichnet, dass man Aroxyketone der Formel

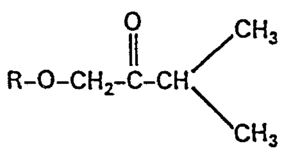

(II)

in welcher
R die oben angegebene Bedeutung hat, mit Pyrimidinyl-halogeniden der Formel

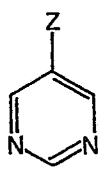

(III)

in welcher
Z für Halogen steht, in Gegenwart eines Verdünnungsmittels und in Gegenwart einer alkalimetall-organischen Verbindung umsetzt und gegebenenfalls anschliessend eine Säure oder ein Metallsalz addiert.

6. Pflanzenwuchsregulierende und fungizide Mittel, gekennzeichnet durch einen Gehalt an mindestens einem Aroxy-pyrimidinylalkanol der Formel (I) bzw. einem Säureadditions-Salz oder Metallsalz-Komplex eines Aroxy-pyrimidinyl-alkanoles der Formel (I).

7. Verfahren zur Regulierung des Pflanzenwachstums sowie zur Bekämpfung von Pilzen, dadurch gekennzeichnet, dass man Aroxy-pyrimidinyl-alkanole der Formel (I) bzw. deren Säureadditions-Salze oder Metallsalz-Komplexe auf die Pflanzen und/oder deren Lebensraum bzw. auf die Pilze und/oder deren Lebensraum ausbringt.

8. Verwendung von Aroxy-pyrimidinyl-alkanolen der Formel (I) bzw. von deren Säureadditions-Salzen oder Metallsalz-Komplexen zur Regulierung des Pflanzenwachstums bzw. zur Bekämpfung von Pilzen.

9. Verfahren zur Herstellung von pflanzenwachstumsregulierenden und fungiziden Mitteln, dadurch gekennzeichnet, dass man Aroxy-pyrimidinyl-alkanole der Formel (I) bzw. deren Säureadditions-Salze oder Metallsalz-Komplexe mit Streckmitteln und/oder oberflächenaktiven Stoffen vermischt.

**Claims**

1. Aroxy-pyrimidinyl-alkanols of the formula

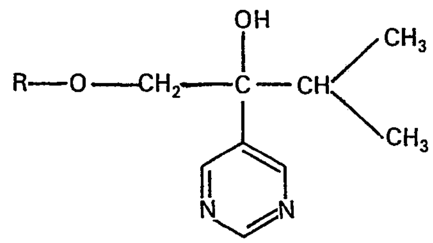

(I)

in which
R represents optionally halogen-substituted naphthyl or the radical of the formula

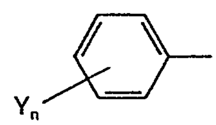

wherein
Y represents fluorine, chlorine, bromine, alkyl with 1 to 4 carbon atoms, cycloalkyl with 5 to 7 carbon atoms, alkoxy with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, alkylthio with 1 to 4 carbon atoms, phenyl which is optionally substituted by halogen and/or alkyl with 1 to 4 carbon atoms, phenoxy which is optionally substituted by halogen and/or alkyl with 1 to 4 carbon atoms, phenylalkoxy which has 1 to 4 carbon atoms in the alkoxy part and is optionally substituted by halogen and/or alkyl with 1 to 4 carbon atoms, or phenylalkyl which has 1 to 4 carbon atoms in the alkyl part and is optionally substituted by halogen and/or alkyl with 1 to 4 carbon atoms, and the index n represents 0, 1, 2 or 3 and wherein the substituents designated by the symbol Y can be identical or different, and acid addition salts and metal salt complexes thereof.

2. Aroxy-primidinyl-alkanol of the formula

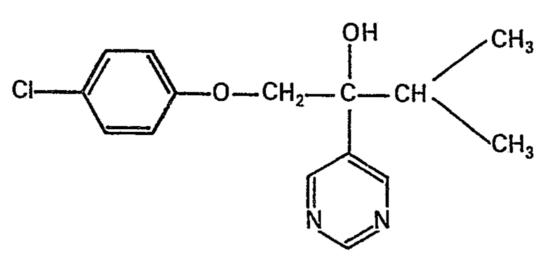

3. Aroxy-pyrimidinyl-alkanol of the formula

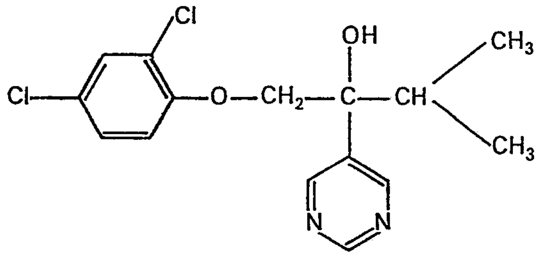

4. Aroxy-pyrimidinyl-alkanol of the formula

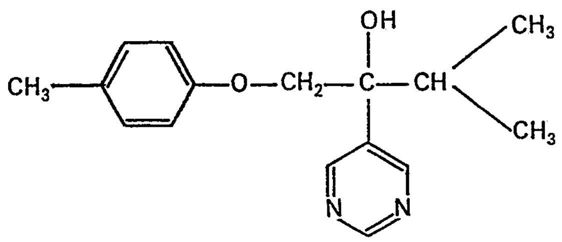

5. Process for the preparation of aroxy-pyrimidinyl-alkanols of the formula

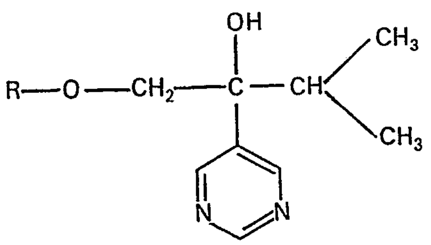

in which
R represents optionally halogen-substituted naphtyl or the radical of the formula

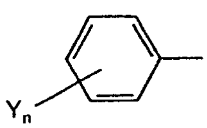

wherein
Y represents fluorine, chlorine, bromine, alkyl with 1 to 4 carbon atoms, cycloalkyl with 5 to 7 carbon atoms, alkoxy with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, alkylthio with 1 to 4 carbon atoms, phenyl which is optionally substituted by halogen and/or alkyl with 1 to 4 carbon atoms, phenoxy which is optionally substituted by halogen and/or alkyl with 1 to 4 carbon atoms, phenylalkoxy which has 1 to 4 carbon atoms, in the alkoxy part and is optionally substituted by halogen and/or alkyl with 1 to 4 carbon atoms, or phenylalkyl which has 1 to 4 carbon atoms in the alkyl part and is optionally substituted by halogen and/or alkyl with 1 to 4 carbon atoms, and the index n represents 0, 1, 2 or 3
and wherein the substituents designated by the symbol Y can be identical or different, and of acid addition salts and metal salt complexes thereof, characterised in that aroxy-ketones of the formula

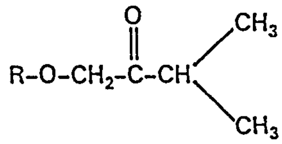

(II)

in which
R has the above mentioned meaning, are reacted with pyrimidinyl halides of the formula

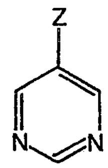

(III)

in which
Z represents halogen, in the presence of a diluent and in the presence of an alkali metal-organic compound, and, if appropriate, an acid or a metal salt is then added on.

6. Plant growth-regulating and fungicidal agents, characterised in that they contain at least one aroxy-pyrimidinyl-alkanol of the formula (I) or an acid addition salt or metal salt complex of an aroxy-pyrimidinyl-alkanol of the formula (I).

7. Method of regulating plant growth and of combating fungi, characterised in that aroxy-pyrimidinyl-alkanols of the formula (I) or acid addition salts of metal salt complexes thereof are applied to the plants and/or their environment or to the fungi and/or their environment.

8. Use of aroxy-pyrimidinyl-alkanols of the formula (I) or of acid addition salts or metal salt complexes thereof for regulating plant growth or combating fungi.

9. Process for the preparation of plant growth-regulating and fungicidal agents, characterised in that aroxy-pyrimidinyl-alkanols of the formula (I) or acid addition salts or metal salt complexes thereof are mixed with extenders and/or surface-active substances.

**Revendications**

1. Aroxy-pyrimidinyl-alcanols de formule:

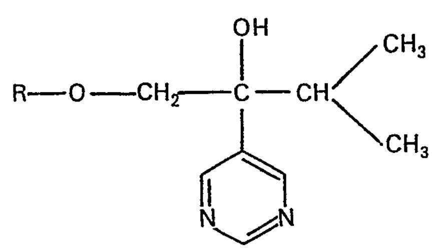

(I)

dans laquelle
R représente un reste naphtyle éventuellement substitué par de l'halogène ou le reste de formule

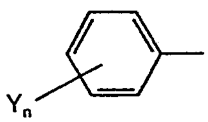

où
Y représente un atome de fluor, de chlore ou de brome, un groupe alkyle ayant 1 à 4 atomes de carbone, cycloalkyle ayant 5 à 7 atomes de carbone, alcoxy ayant 1 à 4 atomes de carbone, halogéno-alkyle ayant 1 ou 2 atomes de carbone et 1 à 5 atomes d'halogène, halogéno-alcoxy ayant 1 ou 2 atomes de carbone et 1 à 5 atomes d'halogène, alkylthio ayant 1 à 4 atomes de carbone, phényle éventuellement substitué par de l'halogène et/ou par un groupe alkyle ayant 1 à 4 atomes de carbone, phénoxy éventuellement substitué par de l'halogène et/ou par un groupe alkyle ayant 1 à 4 atomes de carbone, phénylalcoxy ayant 1 à 4 atomes de carbone dans la partie alcoxy et éventuellement substitué par de l'halogène et/ou par un groupe alkyle ayant 1 à 4 atomes de carbone, phénylalkyle ayant 1 à 4 atomes de carbone dans la partie alkyle et éventuellement substitué par de l'halogène et/ou par un groupe alkyle ayant 1 à 4 atomes de carbone, et l'indice n vaut 0, 1, 2 ou 3, les substituants représentés par le symbole Y pouvant être identiques ou différents, ainsi que leurs sels d'addition d'acide et les complexes formés avec des sels de métaux.

2. Aroxy-pyrimidinyl-alcanol de formule:

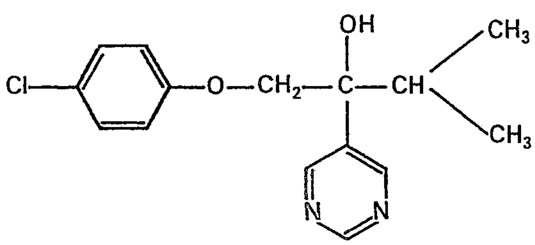

3. Aroxy-pyrimidinyl-alcanol de formule:

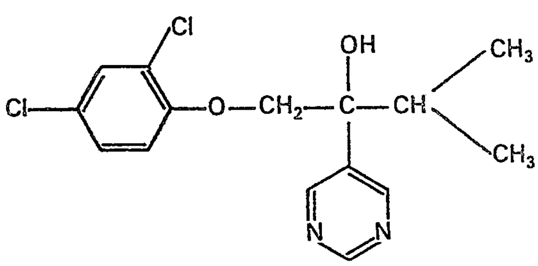

4. Aroxy-pyrimidinyl-alcanol de formule:

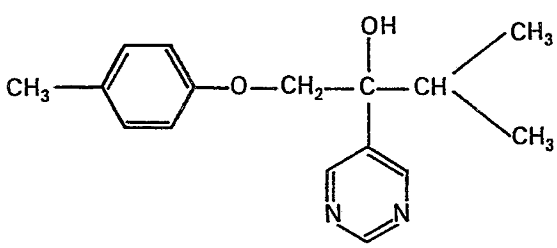

5. Procédé pour préparer des aroxy-pyrimidinyl-alcanols de formule:

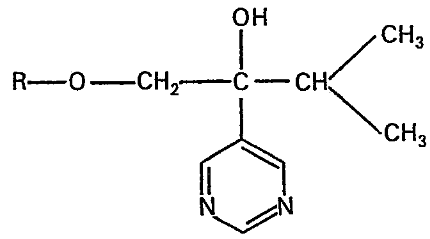 (I)

dans laquelle
R représente un reste naphtyle éventuellement substitué par de l'halogène ou le reste de formule

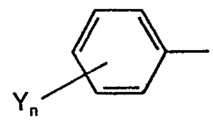

où,
Y représente un atome de fluor, de chlore ou de brome, un groupe alkyle ayant 1 à 4 atomes de carbone, cycloalkyle ayant 5 à 7 atomes de carbone, alcoxy ayant 1 à 4 atomes de carbone, halogéno-alkyle ayant 1 ou 2 atomes de carbone et 1 à 5 atomes d'halogène, halogéno-alcoxy ayant 1 ou 2 atomes de carbone et 1 à 5 atomes d'halogène, alkylthio ayant 1 à 4 atomes de carbone, phényle éventuellement substitué par de l'halogène et/ou par un groupe alkyle ayant 1 à 4 atomes de carbone, phénoxy éventuellement substitué par de l'halogène et/ou par un groupe alkyle ayant 1 à 4 atomes de carbone, phénylalcoxy ayant 1 à 4 atomes de carbone dans la partie alcoxy et éventuellement substitué par de l'halogène et/ou par un groupe alkyle ayant 1 à 4 atomes de carbone, phénylalkyle ayant 1 à 4 atomes de carbone dans la partie alkyle et éventuellement substitué par de l'halogène et/ou par un groupe alkyle ayant 1 à 4 atomes de carbone, et l'indice n vaut 0, 1, 2 ou 3, les substituants représentés par le symbole Y pouvant être identiques ou différents, ainsi que leurs sels d'addition d'acides et leurs complexes avec des sels de métaux, procédé caractérisé en ce qu'on fait réagir des aroxycétones de formule:

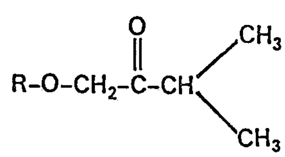 (II)

dans laquelle
R a le sens indiqué ci-dessus, avec des halogénures de pyrimidinyle de formule:

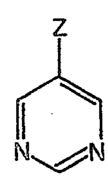 (III)

dans laquelle
Z représente un halogène, en présence d'un diluant et en présence d'un composé organique d'un métal alcalin, et éventuellement ensuite on fixe par addition un acide ou un sel de métal.

6. Substances de croissance et produits fongicides, caractérisés en ce qu'ils contiennent au moins un aroxy-pyrimidinyl-alcanol de formule (I) ou un sel d'addition d'acide ou un complexe, formé avec un sel de métal, d'un aroxy-pyrimidinyl-alcanol de formule (I).

7. Procédé pour réguler la croissance des plantes ainsi que pour combattre des champignons, caractérisé en ce qu'on applique des aroxy-pyrimidinyl-alcanols de formule (I), ou leurs sels d'addition d'acide ou leurs complexes formés avec un sel de métal, sur les plantes et/ou sur leurs biotope ou espace vital ou sur les champignons et/ou leur biotope ou espace vital.

8. Utilisation d'aroxy-pyrimidinyl-alcanols de formule (I) ou de leurs sels d'addition d'acides, ou de leurs complexes formés avec des sels de métaux, pour réguler la croissance des plantes ou pour combattre des champignons.

9. Procédé pour préparer des substances de croissance et des produits fongicides, caractérisé en ce qu'on mélange des aroxy-pyrimidinyl-alcanols de formule (I), ou leurs sels d'addition d'acides, ou leurs complexes formés avec des sels de métaux, avec des agents d'allongement et/ou des substances tensioactives.